Europäisches Patentamt

European Patent Office

Office européen des brevets

(11) Publication number: 0 353 341 A1

# EUROPEAN PATENT APPLICATION

(21) Application number: 88115650.9

(22) Date of filing: 23.09.88

(51) Int. Cl.4: A61N 1/39 , A61B 5/04

(30) Priority: 11.04.88 JP 88626/88

(43) Date of publication of application:
07.02.90 Bulletin 90/06

(84) Designated Contracting States:
DE FR GB IT

(71) Applicant: FUKUDA DENSHI CO., LTD.
39-4, Hongo 3-chome Bunkyo-ku
Tokyo 113(JP)

(72) Inventor: Fukasawa, Eiji Fukuda Denshi Co.,
Ltd.
2-35-8 Hongo Bunkyo-ku
Tokyo(JP)
Inventor: Kawakami, Shigeo Fukuda Denshi
Co., Ltd.
2-35-8 Hongo Bunkyo-ku
Tokyo(JP)
Inventor: Tanaka, Keiji Raionzumanshon 1004
1-4-29 Uenoikenohata Taito-ku
Tokyo(JP)

(74) Representative: Behn, Klaus, Dipl.-Ing.
Patentanwalt Lindenberg 34
D-8134 Pöcking bei München(DE)

(54) Defibrillator electrode.

(57) An electrode for using for the defibrillator, comprising, the first electrode (1) and the second electrode (2) connected to the defibrillator body (3) so as to conduct the high voltage pulse into the living body and lead the electrocardiogram signal from said living body, said first electrode (1) and said second electrode (2) having a first electrode plate (11) and a first grip (12), and a second electrode plate (21) and a second grip (22), each, a first grip (12) and a second grip (12) by which said first electrode plate (11) and a second electrode plate (21) are gripped, respectively, an indicating portion (23) which indicates the wave form of said electrocardiogram signal, mounted on at least one of said first electrode (1) and said second electrode (2).

Fig.2

# ELECTRODE FOR USING FOR THE DEFIBRILLATOR

FBACKGROUND OF THE INVENTION

1. Field of the Invention

The present invention relates to an electrode for using for the defibrillator. More particularly, it relates to an electrode for using for the defibrillator in which the indication portion as a monitor is mounted at a region, on the grip of anyone of two electrodes, where it can be easily seen.

2. Description of the Related Art

Generally, a defibrillator is defined a medical treatment apparatus for putting back to the normal rhythm, the rhythm of a serious arrhythmia composed of standstill, venticular fibrillation, paroxysmal tachycardia and auricular rough fibrillation etc..

It is well known that the heart repeats the fixed normal rhythm of contraction and extention while electric stimulus ,starting from the sinus node inside thereof, extends to each myocardium through the conduction system, and that it thereby functions as the pump of the blood circulatory organs.

However, the above normal rhythm of the heart goes out of order owing to wrong of the above conduction system etc., resulting that the heart is found to fall into the above arrhythmia of standstill, venticular fibrillation, paroxysmal tachycardia, and auricular rough fibrillation.

If the standstill and venticular fibrillation are neglected, the heart stops, which soon results in that a patient suffering from them dies, and therefore it is necessary that they should be treated urgently in particular. Concretely, it removes the arrhythmia to conduct at once into the heart so strong electric stimulus as high voltage pulse from the defibrillator, whereby the heart beat can return back to the normal rhythm.

On the other hand, even if it is unnecessary to treat urgently, there is also a case when medical treatment should be carried out by means of the defibrillator. For example,the avove auricular rough fibrillation can be removed, according to the degree of it. by dosing medicine. However, unless it can be removed by this medicine dose,the treatment should be carred out with the defibrillator, as in the above case of the standstill and venticular fibrillation.

Hereinbefore, the present invention is related to an electrode of the defibrillator used in case of treating heart failure by conducting the high voltage pulse into the heart from outside.

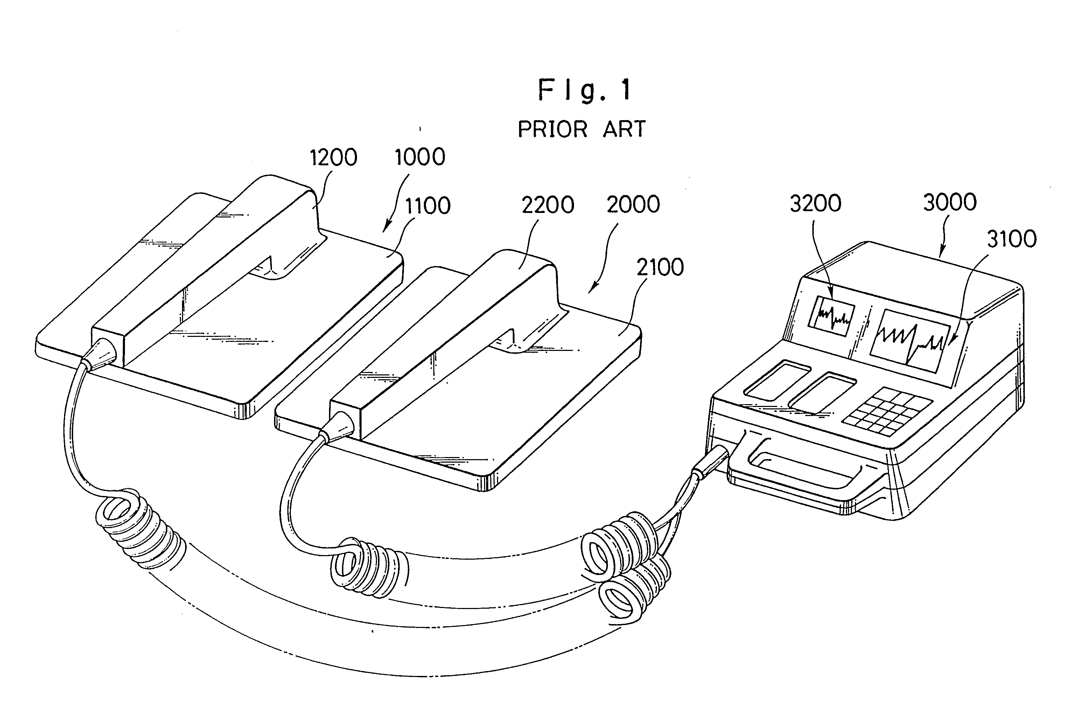

Figure 1 is an explanatory drawing of the conventional electrode for using for the defibrillator.

This conventional electrode consists of a first electrode 1000 and a second electrode 2000 each connected to the defibrillator body 3000,which are constituted by a first electrode plate 1100 and a first grip 1200,and a second electrode plate 2100 and a second grip 2200, respectively.

When the heart of a patient falls into the standstill etc., the doctor contacts closely the electrodes 1000 and 2000 to the surface of the breast of his patient, and he should carry a sequential medical treatment process, as follows.

That is to say,at first, he confirms as to whether or not the heart of his patient falls into the above serious arrhythmia hile he observes a wave form of the electrocardiogram (abbreviate hereinafter to ECG) signal indicated on a monitor 3100.

Next, he conducts the required energized high voltage pulse into the living body of his patient, according to the degree of the confirmed arrhythmia.

Finally, he confirms as to whether or not the arrhythmia has been removed.

However,the prior art has the defect that it is impossible to carry out smoothly the above sequential medical treatment process with the defibrillator. The reason is that it is difficult for the doctor to observe the wave form of the ECG signal indicated on the monitor 3100, because the distance between the doctor and the defibrillator body 3000 is far away.

This defect will be hereinafter explained in more detail.

That is to say, in so urgent case as standstill or venticular fibrillation, it is usual to conduct at once the high voltage pulse into the living body of the patient, because the condition of such a heart failure can be judged according to his completion. However, even if such an urgent case,it is deemed that it had better been desirable to conduct the high voltage pulse, after the the doctor confirms carefully the above condition by observing the wave form by the ECG signal indicated on the monitor 3100.

Moreover, even in so unurgent case as auricular rough fibrillation, it is well known to be to conduct the high voltage pulse with synchronization to R wave of the ECG signal indicated on the monitor 3100.

Therefore, when the doctor treats the heart failure with the defibrillator, at first, it is necessary to confirm the sort of his patient ' s arrhythmia while the doctor observes the wave form of the ECG signal indicated on the monitor 3100.

Also, when the high voltage pulse be conducted into the living body of his patient from the defibrillator body 3000, a shock applied is so strong that his patient's body floats in the air for a moment. Therefore, at second, the doctor must concentrates his attention on pressing the electrodes 1000 and 2000 on the skin surface of his patient's living body with his strong force so as to contact closely these electrodes to the skin surface, before he concentrates his attention on observing the monitor picture.

And, finally, he must confirm as to whether or not the arrhythmia has been removed by the above high voltage pulse conduction, the heart having been able to take back the normal rhythm.

Hereinbefore, the doctor should carry out the above sequential medical treatment process, at the same time that he contacts closely the electrodes 1000 and 2000 to the skin surface of his patient and observes the wave form of the ECG signal indicated on the monitor 3100.

However,as shown in Fig.1, the electrodes 1000 and 2000, and the monitor 3100 mounted on the defibrillator body 3000 are separate. Moreover, generally,these electrodes and the monitor are seted at considerable distance.

Therefore, the distance is far away, between the doctor who should always contact closely said electrodes 1000 and 2000 to the skin surface of the patient, and the monitor 3100, otherwise the angle, wherein the doctor looks at the monitor 3100, is unstable. Thereby, it is often difficult for the doctor to observe the wave form of the ECG signal shown in the monitor 3100.

Hereinbefore, since it is difficult for the doctor to observe the wave form of the ECG signal indicated on the monitor 3100, the prior art has the defect that it is impossible to carry out smoothly so sequential medical treatment process that he confirms the arrhythmia by looking at the wave form, conducts the high voltage pulse with concentration of his attention on pressing the electrodes to the body of the patient,and that he confirms as to whether or not the arrhythmia has been removed with observation of the wave form.

If, at the time of conducting the high voltage pulse, he unreasonably intends to observe the monitor picture, as the body of himself moves, it happens sometimes that the strong force applied on the electrodes 1000 and 2000 weakens.

Consequently, some evils are as follows.

① The value of the contact resistance between the electrode plates 1100 and 2100, and the skin surface of the patient's living body, is larger than the predetermined value.
Thereby, the high voltage pulse of the defibrillator body 3000 is not conducted into the living body, but is consumed as energy on the skin surface.

② The above consumption of energy on the skin surface does injuries, like as serious burn etc., to this skin surface.

③ The treatment time is often prolonged, because it is impossible to carry out smoothly the above sequential medical treatment process, before the setting place of the defibrillator must be restricted in relationship to the position of the patient.

④ In the briefly constructed defibrillator wherein the monitor 3100 or the recorder 3200, as shown in Fig.1, is not mounted, the wave form of the ECG signal could not be confirmed at all. Therefore, the use of such a defibrillator on treatment is sometimes restricted.

SUMMARY OF THE INVENTION

An object of the present invention is to be to carry out smoothly the sequential medical treatment process with the defibrillator, by means of observing easily the wave form of the ECG signal.

The above-mentioned object can be achieved by an electrode for using for the defibrillator comprising, the first electrode and the second electrode connected to the defibrillator body so as to conduct the high voltage pulse into the living body and lead the electrocardiogram signal from said living body, said first electrode and said second electrode having a first electrode plate and a first grip, and a second electrode plate and a second grip, each, said first·grip and said second grip by which said first electrode plate and a second electrode plate are are gripped, respectively, a indicating portion which indicates the wave form of said electrocardiogram signal, mounted on at least one of said first electrode and said second electrode.

BRIEF DESCRIPTION OF THE DRAWINGS

Further features and advantages of the present invention will be apparent from the ensuring description with reference to the accompanying drawings, wherein:

Fig. 1 is an explanatory drawing of the conventional electrode for using for the defibrillator;

Fig. 2 is a whole drawing of an embodiment of the present invention;

Fig. 3 is a drawing of the first embodiment of the present invention;

Fig. 4 is a drawing of the second embodiment of the present invention;

Fig. 5 is a circuit diagram for operating an embodiment of the present invention; and,

Fig. 6 is a drawing of the wave form of the ECG signal indicated on the indicating portion in accordance with the present invention.

## DESCRIPTION OF THE PREFERRED EMBODIMENTS

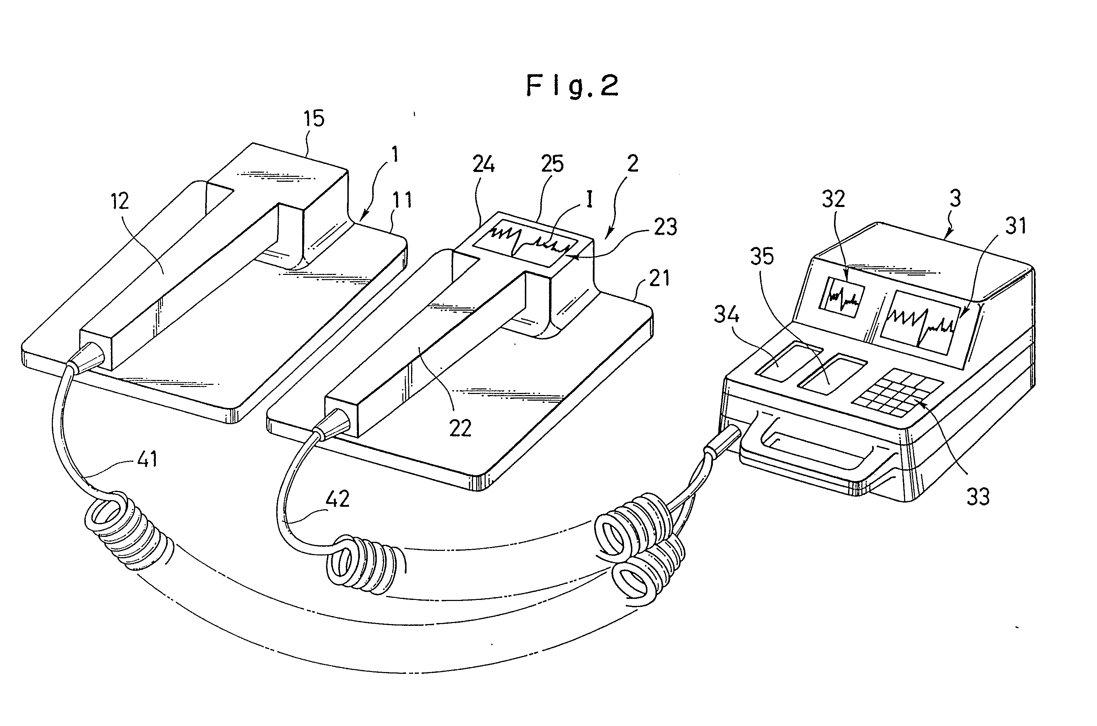

Figure 2 is a whole drawing of an embodiment of the present invention, wherein reference numeral 1 shows a first electrode, 2 a second electrode, 3 a defibrillator body.

Tth first and second electrodes 1 and 2 are respectively connected to the defibrillator body 3 through curled cords 41 and 42, which electrodes 1 and 2 function together the defibrillator electrode conducting the high voltage pulse from the defibrillator body 3 into the living body of the patient, as well as the lead electrode outputting the ECG signal from the living body.

Also,the electrodes 1 and 2 have each a first electrode plate 11 and a second electrode plate 21 as well as a first grip 12 and a second grip 22 by which the first and second electrode plates 11 and 21 are gripped respectively. And, conducting switches 15 and 25, pushed in case of conducting the high voltage pulse, are mounted respectively on the front side of the first and second grips 12 and 22.

Moreover, an indicating portion 23 to indicate the wave I of the ECG signal is mounted on the upper surface, in front of the second grip 22.

The outer shape of the defibrillator body 3 is constructed by a monitor 31, a recorder 32, an operating swich 33, and recesses 34 and 35 receiving each the electrodes 1 and 2, like as the prior art.

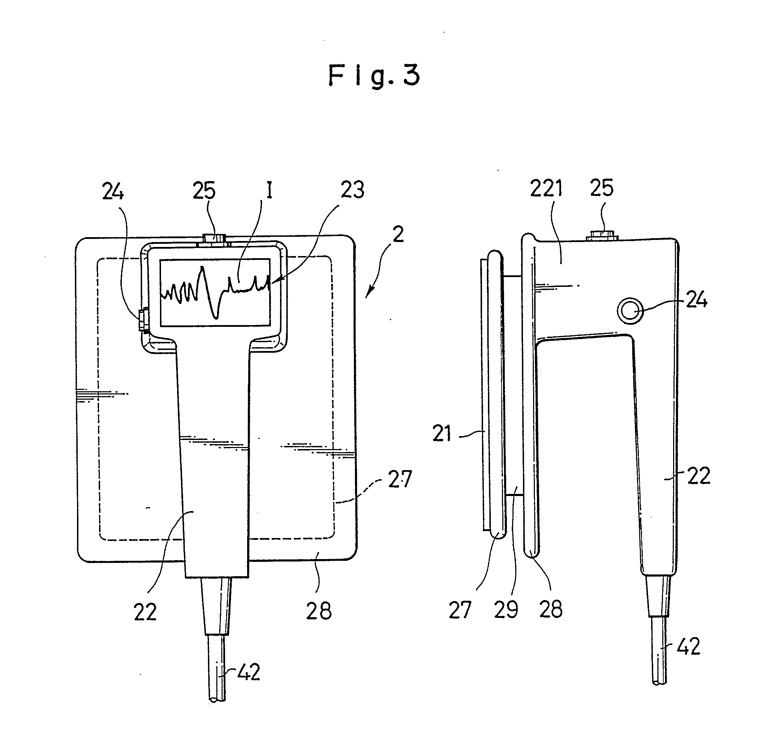

Figure 3 is a drawing of the first embodiment of the present invention, wherein the second electrode 2 is only shown so as to simplify explanation thereof.

The second electrode 2 is made integrally of plastic, in the upper part of which second electrode 2 the second grip 22 elongates to the longitude direction and is formed cylindrically as a whole.

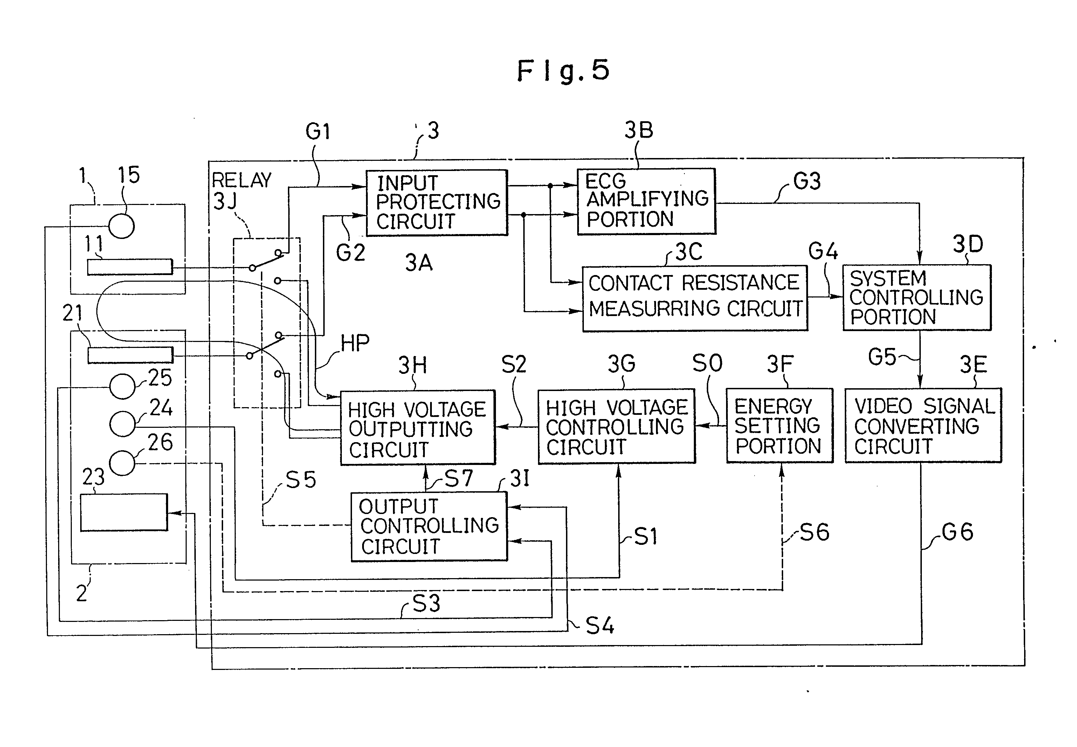

Inside of the second grip 22, there runs through the cirled cord 42 which consists of a cord for connecting the second electrode plate 21 to a high voltage outputting circuit 3 H or an input protecting circuit 3 A according to the operation of a relay 3 J (see Fig.5 ), a cord for connecting the indicating portion 23 to a video signal converting circuit 3 E, a cord for connecting a conducting switch 25 to an output controlling circuit 3 I, a cord for connecting a charging switch 24 to a high voltage controlling circuit 3 G, and a cord for connecting an energy changig swtich 26 to an energy setting portion 3 F.

The second grip 22 bends at the right angle to the down part in the extreme portion thereof, as apparent from Fig.3.

In the extreme portion, the indicating portion 23 to indicate the wave form I of the ECG signal, the conducting switch 25, and the charging switch 24 are mounted on the upper surface, on the front surface, and on the left surface, respectively.

On the top of a portion 221 bending at the right angle, a plate 28, a cylinder 29, and a plate 27 smaller than the plate 28 , are mounted integrally each other as well as they are respectively insulated, for example made of plastic, and on the plate 27 is mounted the second electrode plate 21 so as to be exposed.

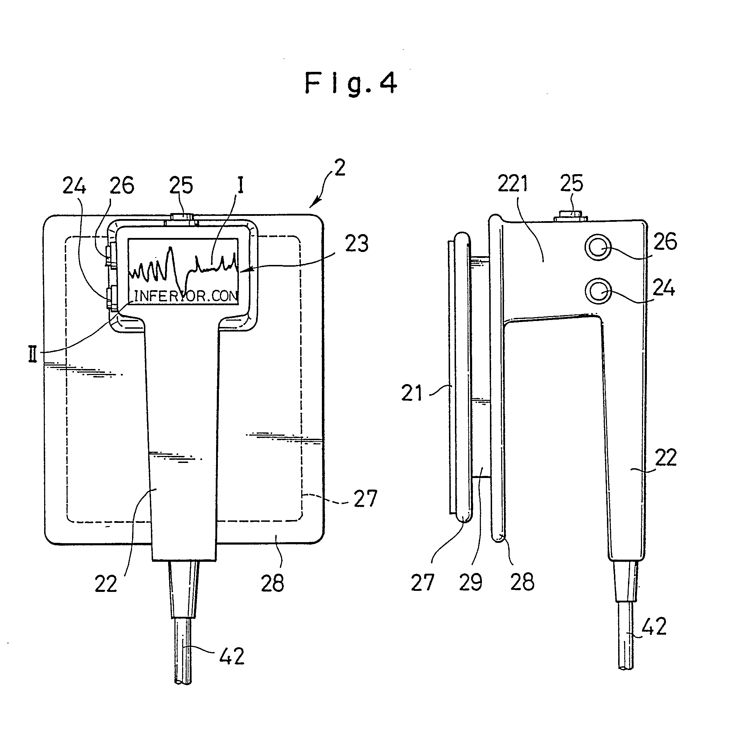

Fig. 4 is a drawing of the second embodiment of the present invention, which second embodiment differs from the first embodiment (see Fig. 3), in that an energy changing switch 26 as well as the charging switch 24 is also mounted on the left surface of the bending portion 221 of the second grip 22, and in that a contact resistance information II is indicated on the indicating portion 23.

The energy changing switch 26 is a switch to change the value of the output energy of the high voltage pulse setted once by the energy setting portion 3 F ( see Fig.5).

The contact resistance information II is an information to mean as to how the contact resistance, between the electrodes 11, 21 and the skin surface of the living body,is at the present time. For example, if the contact resistance is inferior, as shown in Fig. 4, the characters "INFERIOR CON" as the contact resistance information II are indicated on the inditing portion 23.

However, on the contrary, if the contact resistance is good, the characters "GOOD CON" as the contact resistance information II are indicated on the indicating portion 23.

The other first electrode 1 has also the constitution similar to the first and second embodiments of the second electrode 2.

However, with respect to the first electrode 1, that corresponding to the first embodiment of the second electrode 2 (see Fig. 3) has only a conducting switch 15 without the indicating portion 23 and the charging switch 24, moreover that corresponding to the second embodiment of the second electrode 2 (see Fig. 4) has only a conducting switch 15 without the indicating portion 23, the charging switch 24 and the energy changing switch 26.

Fig. 5 is a circuit diagram for operating an embodiment of the present invention.

As shown in Fig. 5, the first electrode 1 and the second electrode 2 have the electrical constitution of the first electrode plate 11 and the conducting switch 15, and of the second electrode plate 21,the indicating portion 23, the charging switch 24, the conducting switch 25 and the energy changing switch 26, respectively.

The defibrillator body 3 consists of the input protecting circuit 3 A to protect an input circuit for the high voltage pulse, the ECG amplifying portion 3 B to amplify an ECG signal, the contact resistance measurring circuit 3 C to measure the contact resistance, the system controlling portion 3 D to control the whole of the apparatus, the video signal converting circuit 3 E to convert the ECG signal and the contact resistance into the video signal so as to indicate them on the indicating portion 23, the energy setting portion 3 F to set previously the output energy (Joule) of the high voltage pulse conducted into the living body, the high voltage controlling circuit 3 G to control the high voltage outputting circuit 3 H in response to the signal from the energy setting portion 3 F and the charging switch 24, the high voltage outputting circuit 3 H to charge a condenser (omitted in Fig. 5) with a high voltage direct current source thereof-(omitted in Fig. 5) in response to the signal from the high voltage controlling circuit 3 G, and to also output the high voltage pulse in response to the signal from the output controlling circuit 3 I, the output controlling circuit 3 I to control the high voltage outputting circuit 3 H and the relay 3 J in response to the signal from the conducting switchs 15 and 25, and the relay 3 J to turn the connection of the electrodes 1 and 2 with the defibrillator body 3 to the side of the high voltage outputting circuit 3 H or that of the ECG amplifying portion 3 B.

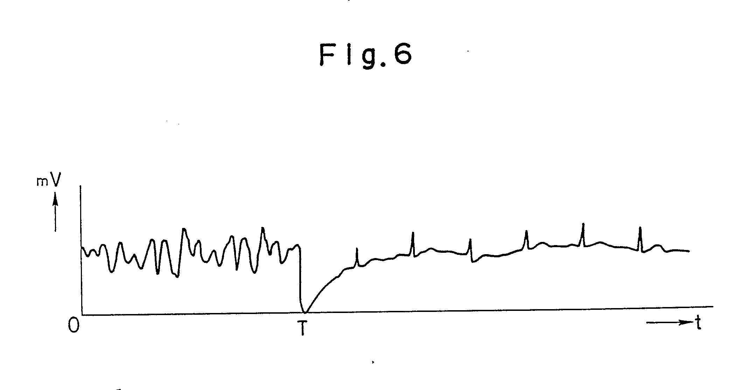

The operation of the embodiments (see Figs. 3 and 4) will be described hereinafter with reference to Fig. 5 and Fig. 6 showing the wave form of the ECG signal.

The operation of the second embodiment shown in Fig. 4 will be only described so as to simplify the description. However, as the first embodiment(see Fig. 3) has the constitution wherein the energy changing switch 26 is removed from the second embodiment, if the operation of the second embodiment be understood, that of the first embodiment will be understood easily.

At first, after the switch of the electric power source (omitted in Fig. 5) is on, when the output energy of the high voltage pulse is setted in the energy setting portion 3 F, and the charging switch 24 is on, the high voltage controlling circuit 3 G operates in response to the signals S 0 and S 1 from the energy setting portion 3 F and the charging switch 24, respectively. Accordingly, a signal S 2 to charge the condenser is output from the high voltage controlling circuit 3 G to the high voltage outputting circuit 3 H, in which the required energy of the high voltage pulse is charged.

After that, when the electrodes 1 and 2 are contacted with the breast of a patient, ECG signals G 1 and G 2 are input, respectively, from the electrode plates 11 and 21 to the input protecting circuit 3 A, through the relay 3 J to be exchanged already on the side of the input protecting circuit 3 A, and then the ECG signals G 1 and G 2 are input to the ECG amplifying portion 3 B.

The ECG amplifying portion 3 B amplifies the difference between the ECG signals G 1 and G 2, and outputs an amplified ECG signal G 3, which is input to the system controlling portion 3 D.

The system controlling portion 3 D treats the amplified ECG signal G 3 and outputs a signal G 5, which is input to the video signal converting circuit 3 E so as to convert the signal G 5 into a video signal G 6.

Accordingly, the amplified ECG signal G 3 is converted into the video signal G 6.

The amplified ECG signal G 3 converted into the video signal G 6 is shown, as the wave form I of the ECG signal, on the indicating portion 23 (see Figs. 2, 3 and 4).

At the same time, the contact resistance information II is also shown on the indicating portion 23, like as the wave form I of the ECG signal,according that the contact resistance value G 4 measurred by the contact resistance measuring circuit 3 C is input to the system controlling portion 3 D in which it is judged as to however the contact resistance is good or inferior.

After it is confirmed that the contact resistance is good in accordance with the contact resistance information II on the indicating portion 23, when the wave form I of the ECG signal is observed (see Fig. 6), the patient ' s heart is founded apparently to fall into venticular fibrillation from the time O through T.

Therefore, at the time T,the conducting switch 15 and 25 are turned on immediately at the same time, whereby conducting signals S 4 and S 3 therefrom are, respectively, input to the output controlling circuit 3 I. Accordingly, a high voltage outputting signal S 7 and a high voltage side exchanging signal S 5 are output from the output controlling circuit 3 I to the high voltage outputting circuit 3 H and the relay 3 J, respectively,whereby the relay 3 J keeps to turn on the high voltage outputting circuit 3 H from 50 to 100 ms,and a high voltage pulse H P with the energy charged ready is conducted from the high voltage outputting circuit 3 H into the living body,through a circuit composed of the electrode 2 as a positive electrode and the electrode 1 as a negative electrode, as shown by the arrow of Fig. 5.

As described hehereinbefore, the high voltage pulse H P is conducted into the living body only for an instant,and thereafter contact points of the relay 3 J automatically returns on the side of the input rotecting circuit 3 A.

Thereby, since the heart gets high energy, the electric potential thereof becomes high, and accordingly a wave form of the ECG signal is widely shown right after the time T, along the vertical axis (mV) of Fig. 6, on the indicating portion 23.

Thereafter,the heart with this high energy returns gradually to the normal rhythm, as apparently from Fig. 6.

If the output energy of the high voltagepulse H P is not enough to returns the heart to the normal rhythm, when the energy changing switch 26 is on, a signal S 6 for changing the value of the output energy is input to the energy setting portion 3 F, and thereby the high voltage pulse H P with larger energy is to be output.

According to the present invention, in the electrode for using for the defibrillator, the indicating portion 23 to show the wave form I of the ECG signal is mounted on at least one of the first electrode 1 and the second electrode 2. Thereby, the doctor , who should treat his patient as contacts the electrodes 1 and 2 to the skin surface of the living body, can observe the wave form of the ECG signal, keeping always himself in the treating posture.

Therefore, the present invention has the primary effect, as follows.

That is to say, it is possible to carry out smoothly so sequential medical treatment process that the doctor confirms the existance of the serious arrhythmia as observes a wave form of the ECG signal, conducts the high voltage pulse into his patient' s living·body while concentrating his attention on pressing the electrodes to the the breast of his patient,and that he confirms , with observation of the wave form, as to whether or not the arrhythmia has been removed .

Moreover,since the doctor can observe a wave form of the ECG signal without his movement, the present invention has another effects, as follows.

That is to say, the electrodes are always to contact closely to the living body,and thereby it does not happen that the energy of the high voltage pulse is consumed on the skin surface, which does injuries, like as serious burn etc., to this skin surface, because the contact resistance is inferior. Since the setting place of the defibrillator is not restricted in relationship to the position of the patient, the treatment speed becomes earlier. If the electrode for using for the defibrillator in accordance with the present invention is applied to the defibrillator without monitor, the use for such a defibrillator on treatment becomes to spread.

the first electrode (1) and the second electrode (2) connected to the defibrillator body (3) so as to conduct the high voltage pulse into the living body and lead the electrocardiogram signal from said living body,

said first electrode (1) and said second electrode (2) having a first electrode plate (11) and a first grip (12), and a second electrode plate (21) and a second grip (22), each,

said first grip (21) and said second grip (22) by which said first electrode plate (11) and a second electrode plate (21) are gripped, respectively,

an indicating portion (23) which indicates the wave form ( I) of said electrocardiogram signal, mounted on at least one of said first electrode (1) and said second electrode (2).

2. An electrode for using for the defibrillator according to claim 1, wherein said indicating portion (23) is mounted on at least one of said first grip (12) and said second grip (22).

3. An electrode for using for the defibrillator according to claim 2, wherein an energy changing switch (26) for changing the value of the output energy of said high voltage pulse is mounted on at least one of said first grip (12) and said second grip (22).

## Claims

1. An electrode for using for the defibrillator comprising:

Fig. 1
PRIOR ART

1200  1000
1100

2200  2000
2100

3000
3100

3200

Fig.2

# F I g. 3

# F I g. 4

INFERIOR.CON

Fig.5

Fig.6

| | DOCUMENTS CONSIDERED TO BE RELEVANT | | EP 88115650.9 |
|---|---|---|---|

| Category | Citation of document with indication, where appropriate, of relevant passages | Relevant to claim | CLASSIFICATION OF THE APPLICATION (Int. Cl 4) |
|---|---|---|---|
| X | DE - A1 - 3 436 945 (O.D.A.M.) <br> * Abstract; page 11, last paragraph - page 13, paragraph 1; fig. 1,2 * | 1,2 | A 61 N 1/39 <br> A 61 B 5/04 |
| A | GB - A - 2 000 682 (ANDERSON) <br> * Page 3, lines 11-31; fig. 3 * | 1,3 | |
| A | GB - A - 1 586 973 (CARDIAC RECORDERS) <br> * Page 2, lines 44-62,76-84; fig. 2,4 * | 1,2 | |
| A | US - A - 4 635 639 (HAKALA) <br> * Abstract; column 4, lines 9-57; fig. 1 * | 1,2 | |
| A | US - A - 4 619 265 (MORGAN) <br> * Abstract; fig. 2 * | 1-3 | TECHNICAL FIELDS SEARCHED (Int. Cl 4) |
| A | US - A - 3 946 743 (MILANI) <br> * Abstract; column 1, lines 34-59; fig. 2 * | 1,3 | A 61 N <br> A 61 B |
| A | WO - A1 - 82/00 414 (R2 CORPORATION) <br> * Abstract; fig. 16-18 * | 1,2 | |

The present search report has been drawn up for all claims

| Place of search | Date of completion of the search | Examiner |
|---|---|---|
| VIENNA | 15-09-1989 | NEGWER |